# DEMANDE DE BREVET EUROPEEN

(11) **EP 1 911 440 A1**
(43) Date de publication de la demande: **16.04.2008**
(21) Numéro de dépôt: 07117774.5
(22) Date de dépôt: 02.10.2007
(51) Int. Cl.: A61K 8/891, A61K 8/895, A61Q 1/02

(54) **Composition cosmétique comprenant des élastomères**

(30) Priorité: 12.10.2006 FR 0654214
(71) Demandeur: L'Oréal, 75008 Paris (FR)
(72) Inventeur: Themens, Agnès, 92340, Bourg La Reine (FR); Leuridan, Maïtena, 75005, PARIS (FR)
(74) Mandataire: Chantraine, Sylvie Hélène

(57) **Abrégé**

La présente invention a pour objet une composition cosmétique anhydre solide comprenant au moins un élastomère de silicone non sphérique, et au moins une poudre d'élastomère de silicone enrobée d'une résine de silicone. L'invention a également pour objet un procédé de maquillage comprenant l'application sur les matières kératiniques de ladite composition ainsi que son utilisation pour obtenir un maquillage homogène et présente une bonne tenue de la couleur dans le temps.

## Description

La présente invention a pour objet une composition cosmétique anhydre, notamment sous forme de « coulé à chaud », comprenant des élastomères de silicone et au moins une huile ester hydrocarbonée de faible poids moléculaire. La composition peut être une composition de maquillage ou de soin des matières kératiniques telles que la peau, les paupières, les lèvres, et notamment la peau. L'invention a également pour objet un procédé de maquillage ou de soin des matières kératiniques.

Les compositions de maquillage contenant une phase grasse sont couramment utilisées en cosmétique en raison de leur bonne adhérence à l'épiderme, leur capacité protectrice et leur capacité à former un film imperméable à l'eau. Le maquillage obtenu est confortable et ne dessèche pas la peau.

Les produits anhydres de maquillage se présentent en général sous forme solide, coulées ou compactées, ou encore sous forme de crème. Les compositions sous forme de « coulé à chaud » peuvent être recherchées dans la mesure où elles peuvent être appliquées de façon satisfaisante à l'aide des doigts ou d'un applicateur, à partir d'un conditionnement sous forme de coupelle ou de stick. Elles contiennent généralement des huiles et des cires leur conférant une consistance stable dans le temps.

Toutefois, du fait de leur teneur élevée en corps gras, ce type de produit présente l'inconvénient de laisser sur la peau une sensation de gras, de collant. En outre, de par leur teneur élevée en cires et en particules solides, les compositions coulées présentent des textures dures, difficiles à étaler. Le maquillage obtenu est alors inhomogène.

Pour améliorer l'étalement de ces compositions lors de leur application sur la peau, il est connu d'introduire des huiles, et de diminuer les teneurs en cires et/ou en particules solides afin d'obtenir des textures plus souples. Cependant, l'introduction de ces huiles peut entraîner des problèmes de stabilité des compositions.

Le but de la présente invention est de fournir une composition anhydre solide, en particulier sous la forme d'un produit coulé, qui présente de bonnes propriétés d'étalement permettant notamment à l'utilisatrice de disposer d'un temps suffisant pour appliquer le produit cosmétique sur les matières kératiniques sur la peau lors de son application sans présenter de sensation de gras et de collant à l'application.

Les inventeurs ont mis en évidence qu'en associant, dans une composition cosmétique anhydre solide au moins un élastomère de silicone non sphérique et une poudre d'élastomère de silicone sphérique, il était possible d'obtenir une composition solide stable, présentant une texture onctueuse, qui s'étale bien lors de son application sur la peau, sans conférer de sensation de gras à l'utilisatrice.
Le maquillage obtenu est homogène et présente une bonne tenue de la couleur dans le temps. La composition ne dessèche pas la peau et le dépôt obtenu est confortable pour l'utilisatrice.

La présente invention a donc pour objet, selon un premier aspect, une composition cosmétique anhydre solide comprenant au moins un élastomère de silicone non sphérique, et au moins une poudre d'élastomère de silicone enrobée d'une résine de silicone.

Selon un second aspect, l'invention a pour objet un procédé de maquillage des matières kératiniques, notamment de la peau, comprenant l'application sur lesdites matières kératiniques d'au moins une composition telle que décrite précédemment.

Selon un troisième aspect, l'invention a pour objet l'utilisation d'une composition telle que décrite précédemment pour obtenir un maquillage homogène et présente une bonne tenue de la couleur dans le temps.

La présente invention a encore pour objet une composition cosmétique anhydre solide comprenant :
- au moins un élastomère de silicone répondant au nom INCI « dimethicone /vinyldimethincone crosspolymer »,
- au moins une poudre d'élastomère de silicone répondant au nom INCI « dimethicone / methicone silsesquioxane crosspolymer »,
- éventuellement au moins une huile ester et /ou,
- éventuellement au moins une cire.

Les compositions selon l'invention sont des compositions solides. Par composition solide au sens de la présente demande, on entend une composition qui ne s'écoule pas sous son propre poids.
Les compositions selon la présente invention sont des compositions anhydres.

Par composition anhydre au sens de la présente demande, on entend une composition comprenant moins de 5 % en poids d'eau, par rapport au poids total de la composition, de préférence moins de 2 % en poids d'eau. Plus préférentiellement encore, la composition est exempte d'eau, l'eau n'étant pas ajoutée lors de la préparation de la composition mais correspondant à l'eau résiduelle apportée par les ingrédients mélangés.

### Elastomères de silicone non sphérique

La composition selon l'invention comprend au moins un élastomère de silicone non sphérique. L'élastomère de silicone peut être non émulsionnant ou émulsionnant.

Selon un mode préféré de réalisation, l'élastomère de silicone est non émulsionnant.

Le terme élastomères de silicone "non émulsionnant" définit des élastomères organopolysiloxane ne contenant pas de chaîne hydrophile telle que motifs polyoxyalkylènes ou polyglycérolés.

L'élastomère de silicone non émulsionnant est un organopolysiloxane réticulé élastomère pouvant être obtenu par réaction d'addition réticulation de diorganopolysiloxane contenant au moins un hydrogène lié au silicium et de diorganopolysiloxane ayant des groupements à insaturation éthylénique liés au silicium, notamment en présence de catalyseur platine ; ou par réaction de condensation réticulation déhydrogénation entre un diorganopolysiloxane à terminaisons hydroxyle et un diorganopolysiloxane contenant au moins un hydrogène lié au silicium, notamment en présence d'un organoétain ; ou par réaction de condensation réticulation d'un diorganopolysiloxane à terminaisons hydroxyle et d'un organopolysilane hydrolysable ; ou par réticulation thermique d'organopolysiloxane, notamment en présence de catalyseur organopéroxyde ; ou par réticulation d'organopolysiloxane par radiations de haute énergie telles que rayons gamma, rayons ultraviolet, faisceau électronique.

De préférence, l'organopolysiloxane réticulé élastomère est obtenu par réaction d'addition réticulation (A2) de diorganopolysiloxane contenant au moins deux hydrogènes liés chacun à un silicium, et (B2) de diorganopolysiloxane ayant au moins deux groupements à insaturation éthylénique liés au silicium, notamment en présence (C2) de catalyseur platine, comme par exemple décrit dans la demande EP-A-295886.

En particulier, l'organopolysiloxane peut être obtenu par réaction de diméthylpolysiloxane à terminaisons diméthylvinylsiloxy et de méthylhydrogénopolysiloxane à terminaisons triméthylsiloxy, en présence de catalyseur platine.

Le composé (A2) est le réactif de base pour la formation d'organopolysiloxane élastomère et la réticulation s'effectue par réaction d'addition du composé (A2) avec le composé (B2) en présence du catalyseur (C2).

Le composé (A2) est avantageusement un diorganopolysiloxane ayant au moins deux groupes alkényles inférieur (par exemple en C2-C4) ; le groupe alkényle inférieur peut être choisi parmi les groupes vinyl, allyl, et propényl. Ces groupements alkényles inférieurs peuvent être situés en toute position de la molécule organopolysiloxane mais sont de préférence situés aux extrémités de la molécule organopolysiloxane.

L'organopolysiloxane (A2) peut avoir une structure chaîne ramifiée, chaîne linéaire, cyclique ou réseau mais la structure chaîne linéaire est préférée. Le composé (A2) peut avoir une viscosité allant de l'état liquide à l'état de gomme. De préférence, le composé (A2) a une viscosité d'au moins 100 centistokes à 25 °C.
Les organopolysiloxanes (A2) peuvent être choisi parmi les méthylvinylsiloxanes, les copolymères méthylvinylsiloxane-diméthylsiloxanes, les diméthylpolysiloxanes à terminaisons diméthylvinylsiloxy, les copolymères diméthylsiloxane-méthylphénylsiloxane à terminaisons diméthylvinylsiloxy, les copolymères diméthylsiloxane-diphénylsiloxane-méthylvinylsiloxane à terminaisons diméthylvinylsiloxy, les copolymères diméthylsiloxane-méthylvinylsiloxane à terminaisons triméthylsiloxy, les copolymères diméthylsiloxane-méthylphénylsiloxane-méthylvinylsiloxane à terminaisons triméthylsiloxy, les méthyl(3,3,3-trifluoropropyl)polysiloxane à terminaisons diméthylvinylsiloxy, et les copolymères diméthylsiloxane-méthyl(3,3,3-trifluoropropyl)siloxane à terminaisons diméthylvinylsiloxy.

Le composé (B2) est en particulier un organopolysiloxane ayant au moins 2 hydrogènes liés au silicium dans chaque molécule et est donc le réticulant du composé (A2).
Avantageusement, la somme du nombre de groupements éthyléniques par molécule du composé (A2) et le nombre d'atomes d'hydrogène liés au silicum par molécule du composé (B2) est d'au moins 4.
Le composé (B2) peut être sous toute structure moléculaire, notamment de structure chaîne linéaire, chaîne ramifiée, structure cyclique.
Le composé (B2) peut avoir une viscosité à 25 °C allant de 1 à 50 000 centistokes, notamment pour être bien miscible avec le composé (A).
Il est avantageux que le composé (B2) soit ajouté en une quantité telle que le rapport moléculaire entre la quantité totale d'atomes d'hydrogène liés au silicium dans le composé (B2) et la quantité totale de tous les goupements à insaturation éthylénique dans le composé (A2) aille dans la gamme de 1/1 à 20/1.
Le composé (B2) peut être choisi parmi les méthylhydrogénopolysiloxanes à terminaisons triméthylsiloxy, les copolymères diméthylsiloxane-méthylhydrogénosiloxane à terminaisons triméthylsiloxy, les copolymères cycliques diméthylsiloxane-méthylhydrogénosiloxane.

Le composé (C2) est le catalyseur de la réaction de réticulation, et est notamment l'acide chloroplatinique, les complexes acide chloroplatinique-oléfine, les complexes acide chloroplatinique-alkenylsiloxane, les complexes acide chloroplatinique-dicétone, le platine noir, et le platine sur support.

Le catalyseur (C2) est de préférence ajouté de 0,1 à 1000 parts en poids, mieux de 1 à 100 parts en poids, en tant que métal platine propre pour 1000 parts en poids de la quantité totale des composés (A2) et (B2).

D'autres groupes organiques peuvent être liés au silicium dans les organopolysiloxane (A2) et (B2) décrits précédemment, comme par exemple des groupes alkyles tels que méthyl, éthyl, propyl, butyl, octyl ; des groupes alkyles substitués tels que 2-phényléthyl, 2-phénylpropyl, 3,3,3-trifluoropropyl ; des groupes aryles tels que phényl, tolyl, xylyl ; des groupes aryles substitutés tel que phényléthyl ; et des groupes hydrocarbonés monovalents substitués tels que un groupe époxy, un gropue ester carboxylate, un groupe mercapto.

Selon un mode préféré de réalisation, l'élastomère de silicone non émulsionnant est mélangé avec au moins une huile hydrocarbonée et/ou une huile siliconée pour former un gel. Dans ces gels, l'élastomère non émulsionnant est sous forme de particules non-sphériques.

Comme élastomères non émulsionnants, on peut utiliser ceux vendus sous les dénominations "KSG-6", "KSG-15", "KSG-16", "KSG-18", "KSG-31", "KSG-32", "KSG-33", "KSG-41 ", "KSG-42", "KSG-43", "KSG-44" par la société Shin Etsu, "DC 9040", "DC9041 ", "DC 9509", "DC9505", "DC 9506" par la société Dow Corning, "GRANSIL" par la société Grant Industries, "SFE 839" par la société General Electric.

L'élastomère de silicone non sphérique peut être émulsionnant.
Par élastomère de silicone émulsionnant on entend un élastomère de silicone comprenant au moins une chaîne hydrophile.

L'élastomère de silicone émulsionnant peut être choisi parmi les élastomères de silicone polyoxyalkylénés.

L'élastomère de silicone polyoxyalkyléné est un organopolysiloxane réticulé pouvant être obtenu par réaction d'addition réticulation de diorganopolysiloxane contenant au moins un hydrogène lié au silicium et d'un polyoxyalkylène ayant au moins deux groupements à insaturation éthylénique.

De préférence, l'organopolysiloxane réticulé polyoxyalkyléné est obtenu par réaction d'addition réticulation (A1) de diorganopolysiloxane contenant au moins deux hydrogènes liés chacun à un silicium, et (B1) de polyoxyalkylène ayant au moins deux groupements à insaturation éthylénique, notamment en présence (C1) de catalyseur platine, comme par exemple décrit dans les brevets US5236986 et US5412004.

En particulier, l'organopolysiloxane peut être obtenu par réaction de polyoxyalkylène (notamment polyoxyéthyléne et/ou polyoxypropylène) à terminaisons diméthylvinylsiloxy et de méthylhydrogénopolysiloxane à terminaisons triméthylsiloxy, en présence de catalyseur platine.

Les groupes organiques liés aux atomes de silicium du composé (A1) peuvent être des groupes alkyles ayant de 1 à 18 atomes de carbone, tels que méthyl, éthyl, propyl, butyl, octyl, décyl, dodécyl (ou lauryl), myristyl, cétyl, stéaryl ; des groupes alkyles substitués tels que 2-phényléthyl, 2-phénylpropyl, 3,3,3-trifluoropropyl ; des groupes aryles tels que phényl, tolyl, xylyl ; des groupes aryles substitués tels que phényléthyl ; et des groupes hydrocarbonés monovalents substitués tels qu'un groupe époxy, un groupe ester carboxylate, ou un groupe mercapto.

Le composé (A1) peut ainsi être choisi parmi les méthylhydrogénopolysiloxanes à terminaisons triméthylsiloxy, les copolymères diméthylsiloxane-méthylhydrogénosiloxane à terminaisons triméthylsiloxy, les copolymères cycliques diméthylsiloxane-méthylhydrogénosiloxane, les copolymères diméthylsiloxane-méthylhydrogénosiloxane-laurylméthylsiloxane à terminaisons triméthylsiloxy.

Le composé (C1) est le catalyseur de la réaction de réticulation, et est notamment l'acide chloroplatinique, les complexes acide chloroplatinique-oléfine, les complexes acide chloroplatinique-alkenylsiloxane, les complexes acide chloroplatinique-dicétone, le platine noir, et le platine sur support.

Avantageusement, les élastomères de silicone polyoxyalkylénés peuvent être formés à partir de composés divinyliques, en particulier des polyoxyalkylènes ayant au moins deux groupes vinyliques, réagissant avec des liaisons Si-H d'un polysiloxane.

L'élastomère de silicone polyoxyalkyléné selon l'invention est véhiculé sous forme de gel dans au moins une huile hydrocarbonée et/ou une huile siliconée. Dans ces gels, l'élastomère polyoxyalkyléné est sous forme de particules non-sphériques.

Des élastomères polyoxyalkylénés sont notamment décrits dans les brevets US5236986, US5412004, US5837793, US5811487 dont le contenu est incorporé par référence.
Comme élastomère de silicone polyoxyalkyléné, on peut utiliser ceux commercialisés sous les dénominations "KSG-21 ", "KSG-20", "KSG-30", "KSG-31 ", KSG-32", "KSG-33", "KSG-210", "KSG-310", "KSG-320", "KSG-330", "KSG-340", "X-226146" par la société Shin Etsu, "DC9010", "DC9011 " par la société Dow Corning.

L'élastomère de silicone émulsionnant peut être également choisi parmi les élastomères de silicone polyglycérolés.

L'élastomère de silicone polyglycérolé est un organopolysiloxane réticulé élastomère pouvant être obtenu par réaction d'addition réticulation de diorganopolysiloxane contenant au moins un hydrogène lié au silicium et de composés polyglycérolés ayant des groupements à insaturation éthylénique, notamment en présence de catalyseur platine.

De préférence, l'organopolysiloxane réticulé élastomère est obtenu par réaction d'addition réticulation (A) de diorganopolysiloxane contenant au moins deux hydrogènes liés chacun à un silicium, et (B) de composés glycérolés ayant au moins deux groupements à insaturation éthylénique, notamment en présence (C) de catalyseur platine.

En particulier, l'organopolysiloxane peut être obtenu par réaction de composé polyglycérolé à terminaisons diméthylvinylsiloxy et de méthylhydrogénopolysiloxane à terminaisons triméthylsiloxy, en présence de catalyseur platine.

Le composé (A) est le réactif de base pour la formation d'organopolysiloxane élastomère et la réticulation s'effectue par réaction d'addition du composé (A) avec le composé (B) en présence du catalyseur (C).

Le composé (A) est en particulier un organopolysiloxane ayant au moins 2 atomes d'hydrogène liés à des atomes de silicium distincts dans chaque molécule.

Le composé (A) peut présenter toute structure moléculaire, notamment une structure chaîne linéaire ou chaîne ramifiée ou une structure cyclique.

Le composé (A) peut avoir une viscosité à 25 °C allant de 1 à 50 000 centistokes, notamment pour être bien miscible avec le composé (B).

Les groupes organiques liés aux atomes de silicium du composé (A) peuvent être des groupes alkyles ayant de 1 à 18 atomes de carbone, tels que méthyl, éthyl, propyl, butyl, octyl, décyl, dodécyl (ou lauryl), myristyl, cétyl, stéaryl, ; des groupes alkyles substitués tels que 2-phényléthyl, 2-phénylpropyl, 3,3,3-trifluoropropyl ; des groupes aryles tels que phényl, tolyl, xylyl ; des groupes aryles substitués tels que phényléthyl ; et des groupes hydrocarbonés monovalents substitués tels qu'un groupe époxy, un groupe ester carboxylate, ou un groupe mercapto. De préférence, ledit groupe organique est choisi parmi les groupes méthyl , phényl, lauryl.

Le composé (A) peut ainsi être choisi parmi les méthylhydrogénopolysiloxanes à terminaisons triméthylsiloxy, les copolymères diméthylsiloxane-méthylhydrogénosiloxane à terminaisons triméthylsiloxy, les copolymères cycliques diméthylsiloxane-méthylhydrogénosiloxane, les copolymères diméthylsiloxane-méthylhydrogénosiloxane-laurylméthylsiloxane à terminaisons triméthylsiloxy.

Le composé (B) peut être un composé polyglycérolé répondant à la formule (B') suivante :

CₘH₂ₘ₋₁-O-[Gly]n-CₘH₂ₘ₋₁ (B')

dans laquelle m est un entier allant de 2 à 6, n est un entier allant de 2 à 200, de préférence allant de 2 à 100, de préférence allant de 2 à 50, de préférence n allant de 2 à 20, de préférence allant de 2 à 10, et préférentiellement allant de 2 à 5, et en particulier égal à 3 ; Gly désigne :

-CH₂-CH(OH)-CH₂-O- ou -CH₂-CH(CH₂OH)-O-

Avantageusement, la somme du nombre de groupements éthyléniques par molécule du composé (B) et du nombre d'atomes d'hydrogène liés à des atomes de silicium par molécule du composé (A) est d'au moins 4.

Il est avantageux que le composé (A) soit ajouté en une quantité telle que le rapport moléculaire entre la quantité totale d'atomes d'hydrogène liés à des atomes de silicium dans le composé (A) et la quantité totale de tous les groupements à insaturation éthylénique dans le composé (B) soit compris dans la gamme de 1/1 à 20/1.

Le composé (C) est le catalyseur de la réaction de réticulation, et est notamment l'acide chloroplatinique, les complexes acide chloroplatinique-oléfine, les complexes acide chloroplatinique-alkenylsiloxane, les complexes acide chloroplatinique-dicétone, le platine noir, et le platine sur support.

Le catalyseur (C) est de préférence ajouté de 0,1 à 1000 parts en poids, mieux de 1 à 100 parts en poids, en tant que métal platine propre pour 1000 parts en poids de la quantité totale des composés (A) et (B).

L'élastomère de silicone polyglycérolé selon l'invention est généralement mélangé avec au moins une huile hydrocarbonée et/ou une huile siliconée pour former un gel. Dans ces gels, l'élastomère polyglycérolé est souvent sous forme de particules non-sphériques.

De tels élastomères sont notamment décrits dans la demande de brevet WO2004/024798.

Comme élastomères de silicone polyglycérolés, on peut utiliser ceux vendus sous les dénominations "KSG-710", "KSG-810", "KSG-820", "KSG-830", "KSG-840" par la société Shin Etsu.

Selon un mode préféré de réalisation, la composition selon l'invention comprend au moins une élastomère de silicone non sphérique non émulsionnant mélangé avec au moins une huile hydrocarbonée et/ou une huile siliconée pour former un gel

L'élastomère de silicone non sphérique peut être présent dans la composition selon l'invention en une teneur en matière active allant de 0,01 % à 8 % en poids, par rapport au poids total de la composition, de préférence allant de 2 % à 7 % en poids, et plus préférentiellement allant de 3 % à 6 % en poids.

### Poudre d'élastomère de silicone enrobée d'une résine de silicone

La composition selon l'invention comprend au moins une poudre d'élastomère de silicone enrobée d'une résine de silicone. La poudre d'élastomère de silicone est sphérique.

Avantageusement, l'élastomère de silicone sous forme de poudre est non émulsionnant. Il peut notamment être obtenu par les procédés de synthèse des élastomères non émulsionnants décrits précédemment.

Des organopolysiloxanes élastomères sphériques sont notamment décrits dans les demandes JP-A-61-194009, EP-A-242219, EP-A-295886, EP-A-765656, dont le contenu est incorporé à titre de référence.

Comme poudres d'organopolysiloxanes élastomères, on peut utiliser celles vendues sous les dénominations "Dow Corning 9505 Powder", "Dow Corning 9506 Powder" par la société Dow Corning ; ces poudres ont pour nom INCI : dimethicone/vinyl dimethicone crosspolymer.

La poudre d'élastomère de silicone est enrobée de résine de silicone.

Selon un mode préféré de réalisation, la résine de silicone peut être une résine silsesquioxane, comme décrit par exemple dans le brevet US5538793 dont le contenu est incorporé à titre de référence.
De telles poudre d'élastomères enrobées de résine de silicone sont notamment vendues sous les dénomination "KSP-100", "KSP-1 01 ", "KSP-102", "KSP-103", KSP-104", "KSP-105" par la société Shin Etsu, et ont pour nom INCI : vinyl dimethicone/methicone silsesquioxane Crosspolymer.
De telles poudres répondent au nom INCI dimethicone silsesquioxane crosspolymer, et en particulier vinyl dimethicone/methicone silsesquioxane crosspolymer.

Selon un autre mode particulier de réalisation, les organopolysiloxanes élastomères sous forme de poudres sphériques peuvent être des poudres de silicone hybride fonctionnalisé par des groupes fluoroalkyle, notamment vendues sous la dénomination "KSP-200" par la société Shin Etsu ; des poudres de silicones hybride fonctionnalisé par des groupes phényl, notamment vendues sous la dénomination "KSP-300" par la société Shin Etsu.

Les particules d'élastomère de silicone peuvent avoir une dureté JIS-A inférieure ou égale à 80 (notamment allant de 5 à 80), et de préférence inférieure ou égale à 65 (notamment allant de 5 à 65). La dureté JIS-A est mesurée selon la méthode JIS K 6301 (1995) établie par le Japanese Industrial Standards Comittee.

En particulier, les particules d'élastomère de silicone peuvent avoir une taille moyenne allant de 0,1 à 500 µm, de préférence de 3 à 200 µm et mieux de 10 et 20 µm. Ces particules peuvent être de forme sphérique, plate ou amorphe, et de préférence de forme sphérique.

La poudre d'élastomère de silicone enrobée d'une résine de silicone, en particulier non émulsionnant, de préférence sphérique, peut être présente dans la composition en une teneur allant de 0,5 % à 8 % en poids, par rapport au poids total de la composition, de préférence allant de 1 % à 6 % en poids.

Outre le (ou les) élastomère(s) de silicone non sphérique(s) et la (ou les) poudre(s) d'élastomère de silicone enrobée(s) d'une résine de silicone, la composition selon l'invention peut comprendre un autre élastomère de silicone, notamment une poudre d'élastomère de silicone non enrobée d'une résine de silicone, telles que les "Dow Corning 9505 Powder", "Dow Corning 9506 Powder" par la société Dow Corning décrites précédemment, ou encore celles décrites dans le document JP-A-02-243612, telles que celles vendues sous la dénomination « TREFIL POWDER E-506C » par la société DOW CORNING.

### Huile ester hydrocarbonée de faible poids moléculaire

La composition selon l'invention peut comprendre au moins une huile ester hydrocarbonée de faible poids moléculaire, aussi appelée « ester court ».
Au sens de la présente invention, on entend par huile ester hydrocarbonée de faible poids moléculaire, un ester hydrocarboné comprenant moins de 40 atomes de carbone.
De tels esters, lorsqu'ils sont introduits dans les compositions selon l'invention, permettent d'améliorer encore la facilité d'étalement du produit sur les matières kératiniques.

Les esters conformes à l'invention peuvent être des monoesters, des diesters ou des polyesters et sont plus particulièrement des monoesters c'est-à-dire portant une unique fonction ester. Ces esters peuvent être linéaires, ramifiés ou cycliques, saturés ou insaturés. En particulier, ils sont ramifiés et saturés. Ils peuvent également être volatils
ou non volatils.
En particulier, les esters hydrocarbonés peuvent répondre à la formule RCOOR' dans laquelle RCOO représente un reste d'acide gras comportant de 2 à 28 atomes de carbone, et R' représente une chaîne hydrocarbonée contenant de 1 à 28 atomes de carbone. Plus particulièrement les groupements R et R' sont tels que l'ester correspondant est non volatil.
De manière avantageuse, les esters hydrocarbonés mono- di- ou polyesters, utilisables dans les compositions cosmétiques conformes à l'invention, comprennent moins de 40 atomes de carbone, et plus de 10 atomes de carbone
Ces esters non volatils peuvent notamment être en C₁₀ à C₂₆ et en particulier en C₁₄ à C₂₄. Ils peuvent être choisis parmi des esters des acides en C₂ à C₁₈ et notamment, des alcools en C₂ à C₂₀ ou de polyols en C₂ à C₈ ou de leurs mélanges.
Ils comportent avantageusement moins de 25 atomes de carbone.
Selon un mode de réalisation particulier, lorsque l'ester hydrocarboné comprend moins de 25 atomes de carbone, il n'est pas une huile volatile.
Ainsi, les esters peuvent être choisis parmi une liste non limitative comprenant les esters de l'acide néopentanoïque comme le néopentanoate d'isodécyle, le néopentanoate d'isotridécyle, le néopentanoate d'isostéaryle, le néopentanoate d'octyldocécyle, les esters de l'acide isononanoïque comme l'isononanoate d'isononyle, l'isononanoate d'octyle, l'isononanoate d'isodécyle, l'isononanoate d'isotridécyle, l'isononanoate d'isostéaryle, mais aussi les esters de l'alcool isopropylique, tels que le myristate d'isopropyle, le palmitate d'isopropyle, le stéarate ou l'isostéarate d'isopropyle, l'octanoate de cétyle, l'octanoate de tridécyle, le 4-diheptanoate et le palmitate d'éthyle 2-hexyle, le benzoate d'alkyle, le diheptanoate de polyéthylène glycol, le diéthyl 2-d'hexanoate de propylèneglycol et leurs mélanges. Ledit ester peut être également choisi parmi les esters de synthèse notamment d'acide gras comme l'huile de purcellin, le myristate d'isopropyle, le palmitate d'éthyle, le stéarate d'octyle ; les esters hydroxylés comme le lactate d'isostéaryle, l'hydroxystéarate d'octyle, l'adipate de diisopropyle, les heptanoates, octanoates, décanoates d'alcool gras et leurs mélanges.

Selon un mode de réalisation particulier, l'ester hydrocarboné court utilisé dans la composition cosmétique conforme à la présente invention peut être choisi parmi d'isononanoate d'isononyle, le palmitate d'éthyle 2-hexyle, et leur mélange.
Selon un mode plus préféré de réalisation, la composition selon l'invention comprend au moins deux esters courts de nature différentes, plus préférentiellement encore un mélange d'isononanoate d'isononyle et de palmitate d'éthyle 2-hexyle

Cet ou ces ester(s) hydrocarboné(s) peut(peuvent) être utilisé(s) dans la composition à en une teneur allant de 10 à 60 % en poids par rapport au poids total de la composition, en particulier de 20 à 55 % en poids, et mieux de 30 à 50 % en poids.

### Huiles additionnelles

Outre l'huile ester hydrocarbonée de faible poids moléculaire, la composition selon l'invention peut comprendre une huile additionnelle choisie parmi les huiles volatiles, non volatiles et leur mélange.

Selon un mode préféré de réalisation, la composition selon l'invention peut comprendre au moins une huile non volatile additionnelle, distincte de l'huile ester hydrocarbonée de faible poids moléculaire et de l'huile utilisée pour former un gel d'élastomère non sphérique.

Par "huile non volatile", on entend une huile restant sur la peau à température ambiante et pression atmosphérique au moins plusieurs heures et ayant notamment une pression de vapeur inférieure à 0,13 Pa (0,01 mm de Hg) à température ambiante (25°C).

Comme huile hydrocarbonée non volatile, on peut notamment citer :
- les huiles hydrocarbonées d'origine animale,
- les huiles hydrocarbonées d'origine végétale telles que les triglycérides constitués d'esters d'acides gras et de glycérol dont les acides gras peuvent avoir des longueurs de chaînes variées de C₄ à C₂₄, ces dernières pouvant être linéaires ou ramifiées, saturées ou insaturées ; ces huiles sont notamment des triglycérides d'acide heptanoïque ou d'acide octanoïque, ou bien encore les huiles de germe de blé, de tournesol, de pépins de raisin, de sésame, de maïs, d'abricot, de ricin, de karité, d'avocat, d'olive, de soja, d'amande douce, de palme, de colza, de coton, de noisette, de macadamia, de jojoba, de luzerne, de pavot, de potimarron, de sésame, de courge, de colza, de cassis, d'onagre, de millet, d'orge, de quinoa, de seigle, de carthame, de bancoulier, de passiflore, de rosier muscat ; le beurre de karité ; ou encore les triglycérides des acides caprylique/caprique comme ceux vendus par la société Stéarineries Dubois ou ceux vendus sous les dénominations Miglyol 810^{®}, 812^{®} et 818^{®} par la société Dynamit Nobel,
- les hydrocarbures linéaires ou ramifiés, d'origine minérale ou synthétique tels que la vaseline, les polydécènes, le polyisobutène hydrogéné tel que le Parleam^{®}, le squalane, les huiles de paraffine, et leurs mélanges,
- les esters de synthèse comprenant plus de 40 atomes de carbone,
- les alcools gras liquides à température ambiante à chaîne carbonée ramifiée et/ou insaturée ayant de 12 à 26 atomes de carbone comme l'octyl dodécanol, l'alcool isostéarylique, l'alcool oléique, le 2-hexyldécanol, le 2-butyloctanol, et le 2-undécylpentadécanol,
- les acides gras supérieurs tels que l'acide oléique, l'acide linoléique, l'acide linolénique et leurs mélanges.

Selon un mode préféré de réalisation, la composition selon l'invention peut comprendre au moins une huile de silicone non volatile additionnelle.

Les huiles de silicone non volatiles utilisables dans la composition selon l'invention peuvent être les polydiméthylsiloxanes (PDMS) non volatiles, les polydiméthylsiloxanes comportant des groupements alkyle ou alcoxy pendants et/ou en bouts de chaîne siliconée, groupements ayant chacun de 2 à 24 atomes de carbone, les silicones phénylées comme les phényl triméthicones, les phényl diméthicones, les phényl triméthylsiloxy diphénylsiloxanes, les diphényl diméthicones, les diphényl méthyldiphényl trisiloxanes, et leurs mélanges.

La composition selon l'invention peut également comprendre une huile volatile additionnelle, distincte de l'huile ester hydrocarbonée de faible poids moléculaire et de l'huile utilisée pour former un gel d'élastomère non sphérique.

Par « huile volatile », on entend au sens de l'invention toute huile susceptible de s'évaporer au contact de la peau, à température ambiante et pression atmosphérique.
Les huiles volatiles de l'invention sont des huiles cosmétiques volatiles, liquides à température ambiante, ayant une pression de vapeur non nulle, à température ambiante et pression atmosphérique, allant en particulier de 0,13 Pa à 40.000 Pa (0,001 à 300 mm de Hg) et de préférence allant de 1,3 à 1300 Pa (0,01 à 10 mm de Hg).

L'huile volatile peut être choisie parmi les huiles volatiles hydrocarbonées, les huiles volatiles siliconées, les huiles volatiles fluorées, et leurs mélanges.

On entend par "huile hydrocarbonée", une huile contenant principalement des atomes d'hydrogène et de carbone et éventuellement des atomes d'oxygène, d'azote, de soufre et/ou de phosphore.

Les huiles hydrocarbonées volatiles peuvent être choisies parmi les huiles hydrocarbonées ayant de 8 à 16 atomes de carbone, et notamment les alcanes ramifiés en C₈-C₁₆ comme les isoalcanes en C₈-C₁₆ d'origine pétrolière (appelées aussi isoparaffines) comme l'isododécane (encore appelé 2,2,4,4,6-pentaméthylheptane), l'isohexadécane, et par exemple les huiles vendues sous les noms commerciaux d'Isopars^{®} ou de Permethyls^{®}.

Comme huiles volatiles, on peut aussi utiliser les silicones volatiles, comme par exemple les huiles de silicones linéaires ou cycliques volatiles, notamment celles ayant une viscosité ≤ 5 centistokes (5 x 10⁻⁶ m²/s), et ayant notamment de 2 à 10 atomes de silicium, de préférence de 2 à 7 atomes de silicium, ces silicones comportant éventuellement des groupes alkyle ou alkoxy ayant de 1 à 10 atomes de carbone. Comme huile de silicone volatile utilisable dans l'invention, on peut citer notamment l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane et leurs mélanges.

L'huile volatile fluorée n'a généralement pas de point éclair. Comme huile volatile fluorée, on peut citer le nonafluoroéthoxybutane, le nonafluorométhoxybutane, le décafluoropentane, le tétradécafluorohexane, le dodécafluoropentane, et leurs mélanges.

Selon un mode particulier de réalisation, la composition ne comprend pas d'huile de silicone volatile additionnelle.
Avantageusement, la composition est exempte d'huiles volatiles additionnelles. En particulier, ou comprend moins de 5% en poids d'huiles volatiles, par rapport au poids total de la composition, de préférence moins de 2 % en poids d'eau

### Cires

La composition selon l'invention peut comprendre au moins une cire.

Par "cire", au sens de la présente invention, on entend un composé gras lipophile, solide à température ambiante (25°C) et pression atmosphérique (760 mm Hg, soit 10⁵ Pa), à changement d'état solide/liquide réversible, ayant en particulier une température de fusion supérieure ou égale à 30°C, notamment supérieure ou égale à 55 °C, et pouvant aller jusqu'à 250 °C, notamment jusqu'à 230 °C, et en particulier jusqu'à 120 °C.
En portant la cire à sa température de fusion, il est possible de la rendre miscible aux huiles et de former un mélange homogène microscopiquement, mais en rétablissant la température du mélange à la température ambiante, on obtient une recristallisation de la cire dans les huiles du mélange.
Les valeurs de point de fusion correspondent, selon l'invention, au pic de fusion mesurée à l'aide d'un calorimètre à balayage différentiel (D.S.C.), par exemple le calorimètre vendu sous la dénomination DSC 30 par la société METLER, avec une montée en température de 5 ou 10 °C par minute.
Les cires, au sens de l'invention, peuvent être celles utilisées généralement dans les domaines cosmétiques ou dermatologiques. Elles peuvent notamment être hydrocarbonées, siliconées et/ou fluorées, comportant éventuellement des fonctions ester ou hydroxyle. Elles peuvent être également d'origine naturelle ou synthétique.
A titre illustratif et non limitatif de ces cires, on peut notamment citer :
- la cire d'abeilles, la cire de lanoline, et les cires d'insectes de Chine ; la cire de riz, la cire de Carnauba, la cire de Candellila, la cire d'Ouricury, la cire de fibres de liège, la cire de canne à sucre, la cire du Japon et la cire de sumac; la cire de montan, les cires microcristallines, les cires de paraffine, les ozokérites, la cire de cérésine, la cire de lignite, les cires de polyéthylène, les cires obtenues par la synthèse de Fisher-Tropsch, les esters d'acides gras et les glycérides concrets à 40 °C et notamment à plus de 55 °C,
- les cires obtenues par hydrogénation catalytique d'huiles animales ou végétales ayant des chaînes grasses, linéaires ou ramifiées, en C₈-C₃₂, notamment l'huile de jojoba hydrogénée, l'huile de tournesol hydrogénée, l'huile de ricin hydrogénée, l'huile de coprah hydrogénée et l'huile de lanoline hydrogénée,
- les cires de silicones ou les cires fluorées, et
- leurs mélanges
Selon une variante particulière de l'invention, la cire peut être choisie parmi les cires de carnauba, les cires microcristallines et leurs mélanges.

La (ou les) cire(s) peu(ven)t être présente(s) dans la composition selon l'invention en une teneur allant de 1 à 15 % en poids, par rapport au poids total de la composition, de préférence de 2 à 12% en poids, et mieux de 5 à 9 % en poids.

Outre les cires précédemment décrites, les compositions selon l'invention peuvent également comprendre des corps gras additionnels tels que des corps gras pâteux.

### Les matières colorantes

Selon un mode de réalisation particulier de l'invention, la composition peut comprendre au moins une matière colorante.

On entend par matière colorante au sens de la présente invention, un composé susceptible de produire un effet optique coloré lorsqu'il est formulé en quantité suffisante dans un milieu cosmétique approprié.

La matière colorante peut être notamment choisie parmi les matières colorantes pulvérulentes, les matières colorantes hydro ou lipo solubles, et leur mélange.

Les matières colorantes pulvérulentes peuvent être choisies parmi les pigments, les nacres, les paillettes, et leurs mélanges.

Selon un mode de réalisation de l'invention, la matière colorante peut comprendre au moins une matière colorante pulvérulente choisie parmi les pigments, les nacres et leur mélange.

Les pigments peuvent être choisis parmi les pigments minéraux, les pigments organiques, et les pigments composites (c'est-à-dire des pigments à base de matériaux minéraux et/ou organiques).

Par pigments, il faut comprendre des particules de toute forme, dotées d'un effet optique, minérales ou de synthèse, insolubles dans le milieu de la composition quelle que soit la température à laquelle la composition est fabriquée.

Les pigments peuvent être choisis parmi les pigments monochromes, les laques, les nacres, les pigments à effet optiques, comme les pigments réfléchissants et les pigments goniochromatiques.

Les pigments minéraux peuvent être choisis parmi les pigments d'oxyde métallique, les oxydes de chrome, les oxydes de fer, le dioxyde de titane, les oxydes de zinc, les oxydes de cérium, les oxydes de zirconium, le violet de manganèse, le bleu de prusse, le bleu outremer, le bleu ferrique, et leurs mélanges.

Les pigments organiques peuvent être par exemple:
- le carmin de cochenille,
- les pigments organiques de colorants azoïques, anthraquinoniques, indigoïdes, xanthéniques, pyréniques, quinoliniques, de triphénylméthane, de fluorane ;
- les laques organiques ou sels insolubles de sodium, de potassium, de calcium, de baryum, d'aluminium, de zirconium, de strontium, de titane, de colorants acides tels que les colorants azoïques, anthraquinoniques, indigoïdes, xanthéniques, pyréniques, quinoliniques, de triphénylméthane, de fluorane. Ces colorants comportent généralement au moins un groupe acide carboxylique ou sulfonique *;*
- les pigments mélaniques.

Parmi les pigments organiques, on peut citer les D&C Blue n°4, D&C Brown n° 1, D&C Green n°5, D&C Green n°6, D&C Orange n°4 , D&C Orange n°5 , D&C Orange n°10 , D&C Orange n °11 , D&C Red n°6, D&C Red n°7, D&C Red n °17, D&C Red n°21, D&C Red n°22, D&C Red n°27, D&C Red n°28, D&C Red n°30, D&C Red n°31, D&C Red n°33, D&C Red n°34, D&C Red n°36, D&C Violet n°2, D&C Yellow n°7, D&C Yellow n°8, D&C Yellow n °10, D&C Yellow n°11, FD&C Blue n° 1, FD&C Green n°3, FD&C Red n°40 , FD&C Yellow n°5, FD&C Yellow n°6.

L'agent de traitement hydrophobe peut être choisi parmi les silicones comme les méthicones, les diméthicones, les perfluoroalkylsilanes ; les acides gras comme l'acide stéarique ; les savons métalliques comme le dimyristate d'aluminium, le sel d'aluminium du glutamate de suif hydrogéné, les perfluoroalkyl phosphates, les perfluoroalkyl silanes, les perfluoroalkyl silazanes, les polyoxydes d'hexafluoropropylène, les polyorganosiloxanes comprenant des groupes perfluoroalkylles perfluoropolyéthers, les acides aminés ; les acides aminés N-acylés ou leurs sels ; la lécithine, le trisostéaryle titanate d'isopropyle, et leurs mélanges.
Les acides aminés N-acylés peuvent comprendre un groupe acyle ayant de 8 à 22 atomes de carbones, comme par exemple un groupe 2-éthyl hexanoyle, caproyle, lauroyle, myristoyle, palmitoyle, stéaroyle, cocoyle. Les sels de ces composés peuvent être les sels d'aluminium, de magnésium, de calcium, de zirconium, de zin, de sodium, de potassium. L'acide aminé peut être par exemple la lysine, l'acide glutamique, l'alanine

Le terme alkyl mentionné dans les composés cités précédemment désigne notamment un groupe alkyle ayant de 1 à 30 atomes de carbone, de préférence ayant de 5 à 16 atomes de carbone.
Des pigments traités hydrophobes sont notamment décrits dans la demande EP-A-1086683.

Par « nacre », au sens de la présente demande, on entend des particules colorées de toute forme, irisées ou non, notamment produites par certains mollusques dans leur coquille ou bien synthétisées et qui présentent un effet de couleur par interférence optique.
Comme exemples de nacres, on peut citer les pigments nacrés tels que le mica titane recouvert avec un oxyde de fer, le mica recouvert d'oxychlorure de bismuth, le mica titane recouvert avec de l'oxyde de chrome, le mica titane recouvert avec un colorant organique notamment du type précité ainsi que les pigments nacrés à base d'oxychlorure de bismuth. Il peut également s'agir de particules de mica à la surface desquelles sont superposées au moins deux couches successives d'oxydes métalliques et/ou de matières colorantes organiques.
Les nacres peuvent plus particulièrement posséder une couleur ou un reflet jaune, rose, rouge, bronze, orangé, brun, or et/ou cuivré.
A titre illustratif des nacres pouvant être introduites en tant que pigment interférentiel dans la première composition, on peut citer les nacres de couleur or notamment commercialisées par la société ENGELHARD sous le nom de Brillant gold 212G (Timica), Gold 222C (Cloisonne), Sparkle gold (Timica), Gold 4504 (Chromalite) et Monarch gold 233X (Cloisonne) ; les nacres bronzes notamment commercialisées par la société MERCK sous la dénomination Bronze fine (17384) (Colorona) et Bronze (17353) (Colorona) et par la société ENGELHARD sous la dénomination Super bronze (Cloisonne) ; les nacres oranges notamment commercialisées par la société ENGELHARD sous la dénomination Orange 363C (Cloisonne) et Orange MCR 101 (Cosmica) et par la société MERCK sous la dénomination Passion orange (Colorona) et Matte orange (17449) (Microna) ; les nacres de teinte brune notamment commercialisées par la société ENGELHARD sous la dénomination Nu-antique copper 340XB (Cloisonne) et Brown CL4509 (Chromalite) ; les nacres à reflet cuivre notamment commercialisées par la société ENGELHARD sous la dénomination Copper 340A (Timica) ; les nacres à reflet rouge notamment commercialisées par la société MERCK sous la dénomination Sienna fine (17386) (Colorona) ; les nacres à reflet jaune notamment commercialisées par la société ENGELHARD sous la dénomination Yellow (4502) (Chromalite) ; les nacres de teinte rouge à reflet or notamment commercialisées par la société ENGELHARD sous la dénomination Sunstone G012 (Gemtone) ; les nacres roses notamment commercialisées par la société ENGELHARD sous la dénomination Tan opale G005 (Gemtone) ; les nacres noires à reflet or notamment commercialisées par la société ENGELHARD sous la dénomination Nu antique bronze 240 AB (Timica), les nacres bleues notamment commercialisées par la société MERCK sous la dénomination Matte blue (17433) (Microna), les nacres blanches à reflet argenté notamment commercialisées par la société MERCK sous la dénomination Xirona Silver et les nacres orangées rosées vert doré notamment commercialisées par la société MERCK sous la dénomination Indian summer (Xirona) et leurs mélanges.

Les matières colorantes pulvérulentes peuvent être présentes dans la composition selon l'invention en une teneur allant de 1 à 40% en poids, par rapport au poids total de la composition, de préférence de 5 à 30 % en poids, et plus préférentiellement encore de 10 à 25% en poids.

Les matières colorantes hydro ou lipo solubles peuvent être choisies parmi les colorants liposolubles, les colorants hydrosolubles, et leur mélange.

Les colorants liposolubles sont par exemple le rouge Soudan, le D&C Red n° 17, le D&C Green n° 6, le β-carotène, l'huile de soja, le brun Soudan, le D&C Yellow n° 11, le D&C Violet n° 2, le D&C orange n° 5, le jaune quinoléine, le rocou, les bromoacides.

Les colorants hydrosolubles sont par exemple le jus de betterave, le bleu de méthylène, le caramel.

### Charges

La composition selon l'invention peut également comprendre au moins une charge.

Par charges, il faut comprendre des particules de toute forme, incolores ou blanches, minérales ou de synthèse, insolubles dans le milieu de la composition quelle que soit la température à laquelle la composition est fabriquée.

Les charges peuvent être minérales ou organiques de toute forme, plaquettaires, sphériques ou oblongues, quelle que soit la forme cristallographique (par exemple feuillet, cubique, hexagonale, orthorombique, etc) . On peut citer le talc, le mica, la silice, le kaolin, les poudres de polyamide (Nylon®) , les poudres de poly-β-alanine, les poudres de polyéthylène, les polyméthacrylates de métyle, les poudres de polyuréthane telle que la poudre de copolymère de diisocyanate d'hexaméthylène et de triméthylol hexyl lactone vendue sous les dénominations PLASTIC POWDER D-400 par la société TOSHIKI, les poudres de polymères de tétrafluoroéthylène (Téflon®), la lauroyl-lysine, l'amidon, le nitrure de bore, les copolymères de chlorure de polyvinylidène/acrylonitrile, de copolymères d'acide acrylique, les poudres de résine de silicone, en particulier les poudres de silsesquioxane (poudres de résine de silicone notamment décrites dans le brevet EP 293795 ; Tospearls® de Toshiba, par exemple), les particules de polyorganosiloxanes élastomères, le carbonate de calcium précipité, le carbonate et l'hydro-carbonate de magnésium, l'hydroxyapatite, les microsphères de silice creuses, les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium ; le sulfate de baryum et leurs mélanges.

Selon un mode préféré de réalisation, la composition selon l'invention peut comprendre au moins une charge sphérique autre que la poudre d'élastomère de silicone enrobée de résine, notamment une poudre de polyamide (Nylon®).

### Galéniques

Les compositions anhydres solides selon l'invention peuvent notamment être des compositions sous forme de « coulé à chaud ». Par composition sous forme de « coulé à chaud » au sens de la présente demande, on entend une composition solide obtenue à l'issue du refroidissement d'une composition introduite à l'état de fusion dans un moule. Les compositions peuvent être coulées sous forme d'un stick ou d'un bâton, ou dans une coupelle.

### Additifs

La composition selon l'invention peut comprendre au moins un autre ingrédient cosmétique usuel pouvant être choisi notamment parmi les agents épaississants lipophiles, les antioxydants, les parfums, les conservateurs, les neutralisants, les filtres solaires, les vitamines, les hydratants, les composés auto-bronzants, les actifs antirides, les émollients, les actifs lipophiles, les agents anti-pollution ou anti-radicaux libres, les sequestrants, les agents filmogènes, les tensioactifs non élastomères, les actifs dermorelaxants, les agents apaisants, les agents stimulant la synthèse de macromolécules dermiques ou épidermiques et/ou empêchant leur dégradation, les agents anti-glycation, les agents anti-irritants, les agents desquamants, les agents dépigmentants, anti-pigmentants, ou pro-pigmentants, les inhibiteurs de NO-synthase, les agents stimulant la prolifération des fibroblastes ou des kéranocytes et/ou la différentiation des kéranocytes, les agents agissant sur la microcirculation, les agents agissant sur le métabolisme énergétique des cellules, les agents cicatrisants, et leurs mélanges.

### Protocole de la facilité d'étalement

L'indice de facilité d'étalement de la composition selon l'invention est déterminé selon le protocole de mesure décrit ci-après.

On prépare un support (carré de 40 mm X 40 mm) constitué d'une couche de mousse de néoprène adhésif sur une de ses faces (vendue sous la dénomination RE70X40 212B de la société JOINT TECHNIQUE LYONNAIS IND). Sur la face non adhésive du support on fixe une couronne adhésive ayant un diamètre interne de 20 mm et dont l'épaisseur est d'environ 250 µm. On applique à l'intérieur de la couronne la composition que l'on arase avec une lame de verre pour obtenir un dépôt de la composition d'environ 250 µm d'épaisseur puis on retire la couronne sans temps de séchage.

Le support est ensuite collé par sa face adhésive sur un embout d'un diamètre de 27 mm fixé sur une presse (STATIF MANUEL SV-1 de la société IMADA Co LTD) équipée d'un dynanomètre (DPS-5R de la société IMADA Co LTD).

Sur un papier couché qualité photo (référence EPSON S041061 de 102g/m²), on dessine une bande de 4 cm de largeur et 21 cm de longueur et dans cette bande on dessine 5 cases ayant chacune une longueur de 4,2 cm selon l'axe longitudinal de la bande. Le papier est placé sur le socle de la presse.

Puis on presse le support (comportant l'échantillon de composition) sur la première case de la bande de papier à une force d'environ 1,5 kg exercée pendant 5 secondes. Puis on déplace de manière rectiligne et régulière le papier sur toute la longueur de la bande de telle sorte que le support soit en contact avec toute la longueur de la bande. La vitesse de déplacement de la bande est de l'ordre de 10 cm/s.

On observe alors visuellement la traînée de produit déposée sur la bande de papier. On peut ainsi comparer les propriétés d'étalement de deux compositions en fonction du nombre de cases, de la première à la cinquième, traversées en tout ou partie par l'éventuelle traînée de produit.

Par convention, le trait de séparation entre la case n et la case n+1 fait partie de la case n.

### Mesure de la texture élastique de la composition

On mesure alors sur la composition la force de pénétration à une température 20°C à l'aide d'un texturomètre vendu sous la dénomination TA-XT2 par la société RHEO, équipé du mobile de mesure « Mobile P10 Cylinder ebonite ». La cellule de mesure est programmée à 5 kg (ce qui correspond à la sensibilité de l'appareil qui peut alors mesurer des forces jusqu'à 5 kg). On introduit la composition à l'état de fusion dans une coupelle H377 rectangulaire de longueur 4,7 cm, largeur 3,9 cm et hauteur 0,5 cm, et on laisse refroidir 24h à 20°C (la composition obtenue est donc sous forme de « coulé à chaud ».

Le mobile descend verticalement à une vitesse (appelée pré-vitesse) de 1,0 mm/s. La mesure est débutée lorsque la composition exerce sur le mobile une force de 0,1 g (force de déclenchement).
Le mobile continue alors de descendre verticalement dans la composition à une vitesse de pénétration de 0,5 mm/s, jusqu'à une profondeur de pénétration de 0,5 mm.

On mesure ainsi la force de pénétration F1 (force maximum relevée au cours de la pénétration).
On maintient le mobile à la profondeur de 0,5 mm pendant un temps de relaxation de 35 s. Au bout de 35 s, on mesure F2 qui correspond à la force exercée par la composition à la fin de sa relaxation (au bout de 35 s).
On remonte ensuite le mobile jusqu'à sa position initiale à une vitesse (appelée post-vitesse) de 2,0 mm/s

On fait 1 mesure au centre de la coupelle, et on teste 3 coupelles différentes. On lit sur l'appareil la force de pénétration F1 exprimée en grammes à chaque mesure puis on calcule une moyenne Fm1. On procède de la même manière pour la force de relaxation F2, dont on calcule la force moyenne Fm2 sur les différentes mesures effectuées.

L'élasticité du produit EL est calculée par la relation suivante : EL = (Fm1-Fm2)/Fm1.
Plus la valeur de EL est faible, plus la composition présente une texture élastique.

La présente invention est décrite plus en détails dans les exemples ci-après.

### Exemples 1 et 2 :

On a préparé des fonds de teint « coulés à chaud » ayant les compositions suivantes:

| **Nom chimique** | **Ex. 1** | **Ex. 2** |
|---|---|---|
| Cire de carnauba | 2,6 | 2,4 |
| Cire microcristalline | 5,1 | 4,8 |
| Gel d'élastomère de silicone à 24% en matière active dans une huile polydiméthylsiloxane 6 cSt vendu sous la dénomination KSG-16 par la société Dow Corning | 15,0 | 14,1 |
| Billes de polydiméthylsiloxane réticulé enrobé de résine de polydiméthylsilsesquioxane (92/8) vendu sous la dénomination KSP 100 par la Société Shin Etsu | 2,0 | 1,9 |
| Nylon 12 | 6,0 | 5,7 |
| Dioxyde de titane | 10,20 | 14,2 |
| Oxydes de fer | 3,8 | 4,9 |
| Palmitate d'éthyle 2-hexyle | 8,6 | 8,1 |
| Isononanoate d'isononyle | 32,6 | 30,7 |
| Phényle triméthicone | 9,0 | 8,5 |
| Conservateurs | qs | qs |
| Elasticité EL = (Fm1-Fm2)/Fm1 | 0,64 | 0,58 |

### Exemple 3 (comparatif)

On a préparé un fond de teint « coulé à chaud » hors invention ayant la composition suivante :

| **Nom chimique** | **Ex. 3 comparatif** |
|---|---|
| Cire de carnauba | 2,6 |
| Cire microcristalline | 5,1 |
| Cire de polytétrafluoro éthylène | 7,0 |
| Gel d'élastomère de silicone à 24% en matière active dans une huile polydiméthylsiloxane 6 cSt vendu sous la dénomination KSG-16 par la société Dow Corning | 0 |
| Billes de polydiméthylsiloxane réticulé enrobé de résine de polydiméthylsilsesquioxane (92/8) vendu sous la dénomination KSP 100 par la Société Shin Etsu | 0 |
| Nylon 12 | 16,0 |
| Nano oxyde de titane | 5,0 |
| Oxydes de fer | 14,0 |
| Palmitate d'éthyle 2-hexyle | 6,9 |
| Isononanoate d'isononyle | 30,9 |
| Phényle triméthicone | 7,4 |
| Conservateurs | qs |
| Elasticité EL = (Fm1-Fm2)/Fm1 | 0,73 |

On a comparé, à l'aide du protocole défini précédemment, l'étalement des compositions 1 et 3. L'exemple comparatif 3 présente une traînée moins longue et moins intense que l'exemple 1 selon l'invention. Ceci indique que le fond de teint selon l'invention s'étale mieux que la fond de teint comparatif 3.

## Revendications

1. Composition cosmétique anhydre solide comprenant :
- au moins un élastomère de silicone non sphérique, et
- au moins une poudre d'élastomère de silicone enrobée d'une résine de silicone.

2. Composition selon la revendication précédente, **caractérisée en ce que** l'élastomère de silicone non sphérique est choisi parmi ceux obtenus :
- par réaction d'addition réticulation de diorganosiloxane contenant au moins un hydrogène lié au silicium et d'un polyoxyalkylène ayant au moins deux groupements à insaturation éthylénique;
- par réaction d'addition réticulation de diorganosiloxane contenant au moins un hydrogène lié au silicium et de composés polyglycérolés ayant des groupements à insaturation éthylénique, notamment en présence de catalyseur platine ;
- par réaction d'addition réticulation de diorganosiloxane contenant au moins un hydrogène lié au silicium et de diorganopolysiloxane ayant des groupements à insaturation éthylénique liés au silicium ;
- par réaction de condensation réticulation déhydrogénation entre un diorganopolysiloxane à terminaisons hydroxyle et un diorganopolysiloxane contenant au moins un hydrogène lié au silicium ;
- par réaction de condensation réticulation d'un diorganopolysiloxane à terminaisons hydroxyle et d'un organopolysilane hydrolysable ;
- par réticulation thermique d'organopolysiloxane ;
- par réticulation d'organopolysiloxane par radiations de haute énergie.

3. Composition selon la revendication précédente, **caractérisée en ce que** l'élastomère de silicone non sphérique est obtenu par réaction d'addition réticulation (A) de diorganopolysiloxane contenant au moins deux hydrogènes liés chacun à un silicium, et (B) de diorganopolysiloxane ayant au moins deux groupements à insaturation éthylénique liés au silicium, notamment en présence (C) de catalyseur platine.

4. Composition selon l'une quelconque des revendications 2 ou 3, **caractérisée en ce que** l'élastomère de silicone non sphérique est obtenu par réaction de diméthylpolysiloxane à terminaisons diméthylvinylsiloxy et de méthylhydrogénopolysiloxane à terminaisons triméthylsiloxy, en présence de catalyseur platine.

5. Composition selon l'une quelconque des revendications précédentes, **caractérisée en ce que** l'élastomère de silicone non sphérique est en mélange avec au moins une huile hydrocarbonée et/ou une huile siliconée pour former un gel.

6. Composition selon l'une quelconque des revendications précédentes, **caractérisée en ce que** l'élastomère de silicone non sphérique est présent dans la composition en une teneur en matière active allant de 0,01 % à 8 % en poids, par rapport au poids total de la composition, de préférence allant de 2 % à 7 % en poids, et plus préférentiellement allant de 3 % à 6 % en poids.

7. Composition selon l'une quelconque des revendications précédentes, **caractérisée en ce que** l'élastomère de silicone enrobé d'une résine est choisi parmi ceux obtenus :
- par réaction d'addition réticulation de diorganosiloxane contenant au moins un hydrogène lié au silicium et de diorganopolysiloxane ayant des groupements à insaturation éthylénique liés au silicium ;
- par réaction de condensation réticulation déhydrogénation entre un diorganopolysiloxane à terminaisons hydroxyle et un diorganopolysiloxane contenant au moins un hydrogène lié au silicium ;
- par réaction de condensation réticulation d'un diorganopolysiloxane à terminaisons hydroxyle et d'un organopolysilane hydrolysable ;
- par réticulation thermique d'organopolysiloxane ;
- par réticulation d'organopolysiloxane par radiations de haute énergie.

8. Composition selon l'une quelconque des revendications précédentes, **caractérisée par le fait que** l'élastomère de silicone enrobé d'une résine est obtenu par réaction d'addition réticulation (A) de diorganopolysiloxane contenant au moins deux hydrogènes liés chacun à un silicium, et (B) de diorganopolysiloxane ayant au moins deux groupements à insaturation éthylénique liés au silicium, notamment en présence (C) de catalyseur platine.

9. Composition selon l'une quelconque des revendications précédentes, **caractérisée par le fait que** l'élastomère de silicone enrobé d'une résine est obtenu par réaction de diméthylpolysiloxane à terminaisons diméthylvinylsiloxy et de méthylhydrogénopolysiloxane à terminaisons triméthylsiloxy, en présence de catalyseur platine.

10. Composition selon l'une quelconque des revendications précédentes, **caractérisée par le fait que** l'élastomère de silicone enrobé d'une résine est un élastomère non émulsionnant.

11. Composition selon l'une quelconque des revendications précédentes, **caractérisée par le fait que** la poudre l'élastomère de silicone est enrobée de résine silsesquioxane.

12. Composition selon l'une quelconque des revendications précédentes, **caractérisée par le fait que** la poudre l'élastomère de silicone enrobé d'une résine est une poudre sphérique.

13. Composition selon l'une quelconque des revendications précédentes, **caractérisée par le fait que** la poudre l'élastomère de silicone enrobé d'une résine de silicone est présente en une teneur allant de 0,5 % à 8 % en poids, par rapport au poids total de la composition, de préférence allant de 1 % à 6 % en poids.

14. Composition selon l'une quelconque des revendications précédentes, **caractérisée en ce qu'**elle comprend au moins une huile ester hydrocarbonée de faible poids moléculaire, notamment comprenant moins de 40 atomes de carbone, et de préférence moins de 25 atomes de carbone.

15. Composition selon la revendication précédente, **caractérisée en ce que** ladite huile ester hydrocarbonée est un monoester répondant à la formule RCOOR' dans laquelle RCOO représente un reste d'acide gras comportant de 2 à 28 atomes de carbone et R' représente une chaîne hydrocarbonée contenant de 1 à 28 atomes de carbone.

16. Composition selon l'une quelconque des revendications 14 ou 15, **caractérisée en ce que** l'huile ester hydrocarbonée comprend au moins 10 atomes de carbone.

17. Composition selon l'une quelconque des revendications 14 à 16, **caractérisée en ce que** l'huile ester hydrocarbonée n'est pas une huile volatile

18. Composition selon l'une quelconque des revendications 14 à 17, **caractérisée en ce que** ledit ester hydrocarboné est choisi parmi les esters de synthèse notamment d'acide gras comme l'huile de purcellin, le myristate d'isopropyle, le palmitate d'éthyle, le stéarate d'octyle ; les esters hydroxylés comme le lactate d'isostéaryle, l'hydroxystéarate d'octyle, l'adipate de diisopropyle, les heptanoates, octanoates, décanoates d'alcool gras, les esters de l'acide néopentanoïque comme le néopentanoate d'isodécyle, le néopentanoate d'isotridécyle, le néopentanoate d'isostéaryle, le néopentanoate d'octyldocécyle, les esters de l'acide isononanoïque comme l'isononanoate d'isononyle, l'isononanoate d'octyle, l'isononanoate d'isodécyle, l'isononanoate d'isotridécyle, l'isononanoate d'isostéaryle, les esters de l'alcool isopropylique, tels que le myristate d'isopropyle, le palmitate d'isopropyle, le stéarate ou l'isostéarate d'isopropyle, l'octanoate de cétyle, l'octanoate de tridécyle, le 4-diheptanoate et le palmitate d'éthyl-2-hexyle, le benzoate d'alkyle, le diheptanoate de polyéthylène glycol, le diéthyl 2- d'hexanoate de propylèneglycol et leurs mélanges.

19. Composition selon l'une quelconque des revendications 14 à 18, **caractérisée en ce qu'**elle comprend au moins deux ester hydrocarbonés de nature différente, et en particulier sont l'isononanoate d'isononyle et le palmitate d'éthyle 2-hexyle.

20. Composition selon l'une quelconque des revendications précédentes, **caractérisée par le fait qu'**elle comprend au moins une huile additionnelle distincte de l'huile ester hydrocarbonée de faible poids moléculaire et de l'huile utilisée pour former un gel d'élastomère non sphérique.

21. Composition selon la revendication précédente, **caractérisée en ce que** ladite huile additionnelle est une huile de silicone non volatile.

22. Composition selon l'une quelconque des revendications précédentes, **caractérisée par le fait qu'**elle comprend au moins une cire.

23. Composition selon la revendication précédente, **caractérisée en ce que** ladite cire est choisie parmi :
- la cire d'abeilles, la cire de lanoline, et les cires d'insectes de Chine ; la cire de riz, la cire de Carnauba, la cire de Candellila, la cire d'Ouricury, la cire de fibres de liège, la cire de canne à sucre, la cire du Japon et la cire de sumac; la cire de montan, les cires microcristallines, les cires de paraffine, les ozokérites, la cire de cérésine, la cire de lignite, les cires de polyéthylène, les cires obtenues par la synthèse de Fisher-Tropsch, les esters d'acides gras et les glycérides concrets à 40 °C et notamment à plus de 55 °C,
- les cires obtenues par hydrogénation catalytique d'huiles animales ou végétales ayant des chaînes grasses, linéaires ou ramifiées, en C₈-C₃₂, notamment l'huile de jojoba hydrogénée, l'huile de tournesol hydrogénée, l'huile de ricin hydrogénée, l'huile de coprah hydrogénée et l'huile de lanoline hydrogénée,
- les cires de silicones ou les cires fluorées, et
- leurs mélanges.

24. Composition selon l'une quelconque des revendications 22 ou 23, **caractérisée en ce que** la cire est choisie parmi les cires de carnauba, les cires microcristallines et leurs mélanges.

25. Composition selon l'une quelconque des revendications 22 à 24, **caractérisée en ce que** la (ou les) cire(s) est(sont) présente(s) en une teneur allant de 1 à 15 % en poids, par rapport au poids total de la composition, de préférence de 2 à 12% en poids, et mieux de 5 à 9 % en poids.

26. Composition selon l'une quelconque des revendications précédentes, **caractérisée en ce qu'**elle comprend au moins une matière colorante choisie parmi les pigments, les nacres, les paillettes et leur mélange.

27. Composition selon l'une quelconque des revendications précédentes, **caractérisée en ce qu'**elle comprend au moins une charge sphérique, distincte de la poudre d'élastomère de silicone enrobée de résine.

28. Composition selon l'une quelconque des revendications précédentes, **caractérisée en ce qu'**elle se présente sous forme de compositions « coulée à chaud ».

29. Composition cosmétique anhydre solide comprenant :
- au moins un élastomère de silicone répondant au nom INCI « dimethicone /vinyldimethincone crosspolymer »,
- au moins une poudre d'élastomère de silicone répondant au nom INCI « dimethicone / methicone silsesquioxane crosspolymer ».

30. Composition selon la revendication précédente, **caractérisée en ce qu'**elle comprend en outre au moins un ester hydrocarboné selon l'une des revendications 16 à 19 et/ou au moins une cire selon l'une des revendications 22 à 24.

31. Procédé de maquillage des matières kératiniques, notamment de la peau, comprenant l'application sur lesdites matières kératiniques d'au moins une composition selon l'une quelconque des revendications précédentes.

32. Utilisation d'une composition selon l'une quelconque des revendications 1 à 31, pour obtenir un maquillage homogène et présente une bonne tenue dans le temps de la couleur.
